# EUROPEAN PATENT APPLICATION

(11) **EP 4 635 620 A1**
(43) Date of publication of application: **22.10.2025**
(21) Application number: 25171198.2
(22) Date of filing: 17.04.2025
(51) Int. Cl.: B01J 13/04, C08J 9/20

(54) **LIGNIN-BASED EXPANDABLE MICROSPHERES**

(30) Priority: 18.04.2024 EP 24171007; 17.05.2024 EP 24176673
(71) Applicant: Nouryon Chemicals International B.V., 1101 BZ Amsterdam (NL)
(72) Inventor: WIJTMANS, Roel, 1101 BZ Amsterdam (NL); ANDREASSON, Bo, 1101 BZ Amsterdam (NL)
(74) Representative: Ingrassia, Fisher & Lorenz UK Ltd.

(57) **Abstract**

The invention relates to lignin-based thermally expandable microspheres, and also to a process for their production. The thermally expandable microspheres comprise a polymeric shell surrounding a hollow core, wherein the hollow core comprises a blowing agent, and the polymeric shell comprises isolated lignin.

## Description

### Technical Field

The invention relates to lignin-based thermally expandable microspheres, and also to a process for their production.

### Background Art

Thermally expandable microspheres are known in the art, and are described for example in US3615972, WO 00/37547 and WO2007/091960. A number of examples are sold under the trade name Expancel^{®}. They can be expanded to form extremely low weight and low density fillers, and find use in applications such as foamed or low density resins, paints and coatings, cements, inks and crack fillers. Consumer products that often contain expandable microspheres include lightweight shoe soles (for example for running shoes), textured coverings such as wallpaper, solar reflective and insulating coatings, food packaging sealants, wine corks, artificial leather, foams for protective helmet liners, and automotive weather strips.

Thermally expandable polymer microspheres usually comprise a thermoplastic polymeric shell, with a hollow core comprising a blowing agent which expands on heating. Examples of blowing agents include low boiling hydrocarbons or halogenated hydrocarbons, which are liquid at room temperature, but which vapourise on heating. To produce expanded microspheres, the expandable microspheres are heated, such that the thermoplastic polymeric shell softens, and the blowing agent vapourises and expands, thus expanding the microsphere. Typically, the microsphere diameter can increase between 1.5 and 8 times during expansion. Expandable microspheres are marketed in various forms, e.g. as dry free-flowing particles, as aqueous slurry or as a partially dewatered wet cake.

Expandable microspheres can be produced by polymerizing ethylenically unsaturated monomers in the presence of a blowing agent, for example using a suspension-polymerisation process. Typical monomers include those based on acrylates, acrylonitriles, acrylamides, vinylidene dichloride and styrenes. A problem associated with such thermoplastic polymers is that they are typically derived from petrochemicals, and are not derived from sustainable sources. In addition, many polymers are non-biodegradeable, or at least biodegrade so slowly that they risk cumulative build-up in the environment. However, it is not necessarily easy merely to replace the monomers with more sustainable-derived alternatives, since it is necessary to ensure that acceptable expansion performance is maintained. For example, the polymer must have the right surface energy to get a core-shell particle in a suspension polymerization reaction so that the blowing agent is encapsulated. In addition, the produced polymer must have good gas barrier properties to be able to retain the blowing agent. Further, the polymer must have suitable viscoelastic properties above glass transition temperature T_{g} so that the shell can be stretched out during expansion. Therefore, replacement of conventional monomers by bio-based monomers is not easy.

Expandable microspheres have been described, in which at least a portion of the monomers making up the thermoplastic shell are bio-based, being derivable from renewable sources. For example, WO2019/043235 describes polymers comprising lactone monomers, WO2019/101749 describes copolymers comprising itaconate dialkylester monomers, and WO2020/099440 as well as WO2021/234010 A1 disclose thermally expandable microspheres made from cellulose-based biopolymers.

However, it has been found that these bio-based expandable microspheres may have some drawbacks from a commercial point of view, such as reduced commercial availability of the raw material and a higher price of the raw material compared to fossil-fuel based raw material, but also from a viewpoint of performance of the expandable microspheres, such as sometimes unsatisfactory and less flexible expansion characteristics which might not always be exactly tuneable for a particular desired application. Moreover, there is also always room for improvement as regards biodegradability of the polymer shell. Moreover, it has been found that such bio-based expandable microspheres tend to be flammable. This may impose complications for the use of such microspheres in applications with high requirements as regard fire safety.

Hence, there remains a need for alternative thermoplastic expandable microspheres in which the thermoplastic polymer shell is, at least in part, derived from sustainable sources. Moreover, there further remains a need for providing expandable microspheres in which the thermoplastic polymer shell is, at least in part, derived from sustainable sources and wherein the expandable microspheres have satisfactory expansion characteristics which even more preferably are tunable according to needs. It would be further desirable if such alternative thermally expandable microspheres also have improved flammability resistance, in particular compared to known and commercially available thermally expandable microspheres. The present invention is, therefore, directed to finding improved bio-based thermally expandable polymeric microspheres having at least some of the aforementioned desirable properties.

### Summary of Invention

The present invention is directed to thermally expandable microspheres comprising a polymeric shell surrounding a hollow core, wherein the hollow core comprises a blowing agent, and the polymeric shell comprises isolated lignin. The thermally expandable microspheres described herein solve the above described problems.

The invention is also directed to a method for preparing thermally expandable microspheres comprising a polymeric shell surrounding a hollow core, wherein the hollow core comprises a blowing agent, and the polymeric shell comprises isolated lignin, the method comprising the following steps: (i) preparing a mixture comprising the isolated lignin and the blowing agent in a solvent; and (ii) spray-drying the mixture obtained in step (i) so to obtain thermally expandable microspheres.

### Brief Description of Drawings

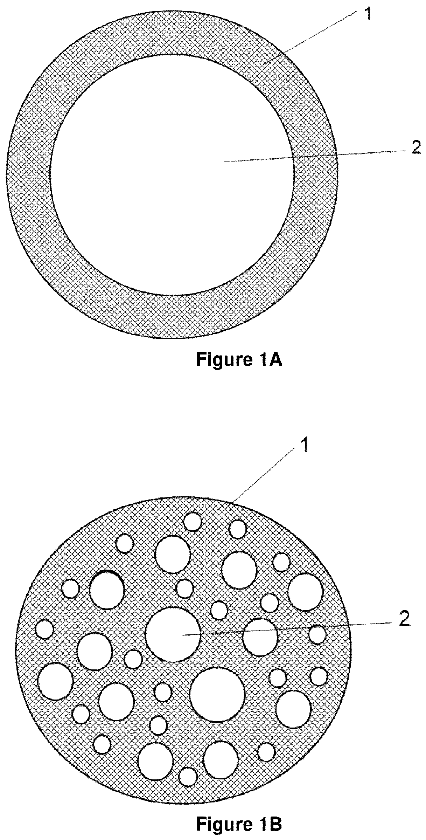

Figure 1 illustrates the difference between a single core (Figure 1A) and multi-core (Figure 1B) microsphere.

### Description of Embodiments

One aspect of the present invention are thermally expandable microspheres comprising a polymeric shell surrounding a hollow core, wherein the hollow core comprises a blowing agent, and the polymeric shell comprises isolated lignin.

The expandable microspheres are based on a polymeric shell comprising isolated lignin. Lignin is a class of complex organic polymers that form key structural materials in the support tissues of most plants, in particular of trees. In other words, lignin is embedded in and part of a complex natural matrix, often in the form of lignocellulose, wherein the lignin is bonded to hemicellulose and/or cellulose. Lignins are particularly important in the formation of cell walls, especially in wood and bark, because they lend rigidity and do not rot easily. From a chemical point of view, lignins are polymers made by crosslinking phenolic precursors. The expandable microspheres of the present invention comprise isolated lignin. The term "isolated lignin" means that the lignin has been isolated from its natural matrix, such as from lignocellulose, for instance by chemical and/or physical (such as mechanical) processes. In other words, the term "isolated lignin" as used herein is used to differentiate the isolated lignin as used in the expandable microspheres of the present invention from lignin which is still contained in its natural matrix.

Lignin can be isolated from its natural matrix by various physical and chemical processes so to yield the isolated lignin used in the present invention. These processes are commonly known by the skilled person and isolated lignin obtained from any of these commonly known processes can be used in the present invention. The isolated lignin can be lignin as such which has merely been removed from its natural matrix or lignin which has been chemically modified during or after the removal from its natural matrix.

In a preferred embodiment the isolated lignin is chemically modified lignin. Chemical modification of lignin can include a derivatization of the lignin, for instance by adding functional groups to the lignin, such as sulfur-containing functional groups, or also merely by cleaving bonds in the lignin to reduce the molecular weight thereof.

Commonly known types of isolated lignin are Kraft lignin, sulfonated lignin, organosolv lignin, hydrolysis lignin, steam explosion lignin, milled wood lignin, and soda lignin. Hence, in an embodiment, the lignin comprises a lignin selected from the group consisting of Kraft lignin, sulfonated lignin, organosolv lignin, hydrolysis lignin, steam explosion lignin, milled wood lignin, soda lignin, and any combination thereof. In a preferred embodiment, the isolated lignin comprises Kraft lignin, sulfonated lignin, or a combination thereof. Particularly preferred is that the lignin comprises Kraft lignin, such as acetylated Kraft lignin. Isolated lignin can be purchased commercially.

The Kraft process (also known as Kraft pulping or sulfate process) is a process that involves treatment of lignin containing organic material, such as wood, usually in the form of chips, with a hot mixture of water, sodium hydroxide, and sodium sulfide that breaks the bonds that link lignin, hemicellulose, and cellulose. The process involves several steps, both mechanical and chemical. The Kraft process yields so-called Kraft lignin. The Kraft lignin can be further derivatized, for instance by acetylation so that acetylated Kraft lignin can be obtained.

So-called sulfonated lignin (or also lignosulfonate) may be obtained by the sulfite process. In the sulfite process, lignin containing organic material, such as wood, usually in the form of chips is heated with solutions of sulfite and bisulfite ions. These chemicals cleave the bonds between the cellulose and lignin components of the lignocellulose. During this process the lignin is converted to lignosulfonates.

Organosolv lignin may be obtained using the organosolv pulping process which is a pulping technique that uses an organic solvent to solubilise lignin and hemicellulose. Organosolv pulping involves contacting lignin containing organic material such as wood, for instance in the form of chips, with an aqueous organic solvent at increased temperatures usually ranging from about 140 to about 220 °C. This causes the lignin to break down by hydrolytic cleavage of alpha aryl-ether links into fragments that are soluble in the solvent system. Suitable solvents used in this process include acetone, methanol, ethanol, butanol, ethylene glycol, formic acid, and acetic acid.

Hydrolysis lignin, steam explosion lignin, milled wood lignin, and soda lignin are further types of lignin which may be obtained from lignin containing organic material.

In embodiments, the number average molecular weight (Mₙ) of the isolated lignin used to form the microspheres is in the range of from 500 to 700 000, for example in the range of from 1 000 to 500 000, preferably from 2000 to 400 000. In embodiments, it is in the range of from 1 000 to 100 000, for example from 1 000 to 80 000, or from 2 000 to 50 000. In some embodiments, the number average molecular weight (Mₙ) of the isolated lignin is from 1 000 to 50 000, such as from 1 000 to 30 000, from 1 000 to 10 000, from 1 000 to 8 000, or from 2 000 to 7 000.

The polymeric shell can comprise or consist of one or more polymeric components, in which at least one component, more than one component or all polymeric components are selected from such isolated lignin. In other words, in some embodiments, the polymeric shell comprises more than one, such as two, three or four, different types of isolated lignin. Where there the shell comprises polymers other than those described herein (i.e. isolated lignin), their content is typically up to 50 wt%, for example less than up to 30 wt%, or less than up to 10 wt%, such as 9 wt% or below, 5 wt% or below or even 2 wt% or below. These percentages are based on the total polymer content of the shell. In some embodiments, the shell comprises polymers other than isolated lignin in an amount of at least 0.1 wt%, at least 0.5 wt%, at least 1 wt%, at least 5 wt%, or at least 10 wt%. In some embodiments, the shell comprises polymers other than isolated lignin in an amount of 0.1 to 50 wt%, such as 0.5 to 50 wt%, 1 to 45 wt%, or 5 to 40 wt%. In a preferred embodiment, however, the shell consists only of isolated lignin.

In some embodiments, the polymeric shell can comprise at least 5 wt%, such as at least 10 wt%, at least 20 wt%, or at least 30 wt%, preferably at least 50 wt.%, such as preferably at least 60 wt.%, at least 70 wt.% or at least 80 wt.%, and more preferably at least 90 wt%, such as at least 95 wt% or at least 98 wt% isolated lignin. These percentages are based on the total polymer content of the shell.

The polymers other than those described herein are not limited and, hence, if present, any polymeric component known to the skilled person can be used as a polymer other than those described herein (i.e. isolated lignin). Polymers other than those described herein are also described herein as "further polymeric component(s)". In some embodiments, the polymeric shell further comprises at least one further polymeric component, i.e. a polymeric component which is not isolated lignin. In some embodiments, the polymeric shell further comprises at least one further polymeric component selected from the group consisting of polysaccharides, polysaccharide derivatives, polyesters, polyethers, polyacids, polyols, polyalkenes, or any combination thereof. In some embodiments, the polysaccharides and polysaccharide derivatives may be selected from the group consisting of cellulose, cellulose derivatives, chitosan, hemicellulose, and alginates, and preferably are selected from cellulose, cellulose derivatives, and chitosan. In a preferred embodiment, the further polymeric component is selected from alkyl cellulose, carboxy cellulose, copolymers of vinyl acetate, polylactic acid, polyethylene glycol, polyvinyl alcohol, polyacids, polyacrylic acid, nanocrystalline cellulose, or combination thereof, preferably is selected from alkyl cellulose, carboxy cellulose, copolymers of vinyl acetate, polylactic acid, polyethylene glycol, polyacrylic acid, and combinations thereof, and more preferably is polyethylene glycol, polyacrylic acid, or a combination thereof. Any combination of the aforementioned further polymeric components can be used. Such combinations also include copolymers of any of the aforementioned further polymeric components.

The thermally expandable microspheres are hollow, in which the shell comprises the isolated lignin, and the hollow center or core comprises one or more blowing agents. The isolated lignin used to prepare the microspheres typically have a density of 1.0 - 1.35 g/cm³. In the expanded microspheres, the density is typically less than 1 g/cm³, and is suitably in the range of from 0.005 to 0.8 g/cm³, or from 0.01 to 0.75 g/cm³. In further embodiments, the density of the expanded microspheres is in the range of from 0.01 to 0.5 g/cm³. Higher densities, particularly densities of 1 g/cm³ or more, indicate that the microsphere samples are not suitable for use.

The thermally expandable microspheres preferably have a temperature at which expansion starts, Tₛₜₐᵣₜ, of from 100°C to 200°C. The temperature at which expansion starts is called Tₛₜₐᵣₜ, while the temperature at which maximum expansion is reached is called Tₘₐₓ. Tₛₜₐᵣₜ and Tₘₐₓ may be determined using standard measuring techniques as commonly known by the skilled person. For instance, Tₛₜₐᵣₜ and Tₘₐₓ can be determined in a temperature ramping experiment, by using for example a Mettler-Toledo Thermomechanical Analyser, such as Mettler-Toledo TMA/SDTA 841e, using a heating rate of 20°C / min and a load (net.) of 0.06 N. In such a temperature ramping experiment, a sample of known weight of the thermally expandable microspheres is heated with a constant heating rate of 20°C / min under a load (net.) of 0.06 N. When expansion of the thermally expandable microspheres starts, the volume of the sample increases and moves the load upwards. From such measurement, an expansion thermogram is obtained wherein the ordinate indicates the height of moving the load upwards and the abscissa indicates the temperature. Tₛₜₐᵣₜ and Tₘₐₓ can be determined from this expansion thermogram for instance by using STARe software from Mettler-Toledo.

In embodiments, the thermally expandable microspheres have a Tₛₜₐᵣₜ in the range of from 110°C to 190°C. Even more preferably the thermally expandable microspheres have a Tₛₜₐᵣₜ in the range from 120°C to 185°C, and most preferably in the range of from 125°C to 185°C.

A number of factors can result in high densities, such as poor expansion characteristics, which can arise where too many of the microspheres contain insufficient blowing agent to enable adequate expansion. This can result from the polymer shell being too permeable to the blowing agent, or due to the formation of so-called "multiple core" microspheres where, instead of a single blowing agent-containing core, there are multiple blowing agent-containing cores within the shell (e.g. like a microspherical foam or sponge). In such multi-core microspheres, the blowing agent concentration is typically too low to reduce the density adequately. Another cause is aggregation or agglomeration of the polymer, resulting in poor microsphere production and a denser material. Too high a proportion of aggregated material or poorly expanding microspheres can also lead to large inhomogeneity in the expansion characteristics of the resulting microsphere product. This is particularly unfavourable for surface-sensitive applications such as coatings, where a smooth finish is desirable.

Illustrative cross sections of single core and multi-core microspheres are provided in Figures 1A and 1B respectively, where regions of polymer, 1, are represented by the cross-hatched areas, and blowing agent-containing regions, 2, are represented by blank areas.

In further embodiments, the polymeric shell can comprise particles to improve the mechanical properties and gas barrier of the polymer shell. Examples of such particles are talc, montmorillonite, and various types of clay, such as bentonite.

The hollow core of the thermally expandable microspheres of the present invention comprises a blowing agent. The blowing agent can comprise one or more different blowing agents. The one or more blowing agents generally have a boiling point above 25°C at 5.0 bara pressure or above 25°C at 3.0 bara pressure, where "bara" stands for bar-absolute. In embodiments, they have a boiling point above 25°C at atmospheric pressure (1.013 bara). Typically, they have a boiling point of 250°C or less at atmospheric pressure, for example 220°C or less, or 200°C or less. They are preferably inert, and do not react with the isolated lignin shell. Boiling points at elevated pressures can be calculated using the Clausius Clapeyron equation.

Examples of blowing agents include alcohols, dialkyl ethers, alkanes and halocarbons, e.g. chlorocarbons, fluorocarbons or chlorofluorocarbons. In embodiments, the alcohol comprises a C₂ to C₈ alcohol, such as a C₂ to C₆ alcohol or a C₂ to C₄ alcohol. In embodiments, the dialkyl ether comprises two alkyl groups each selected from C₂ to C₅ alkyl groups. In embodiments, the alkane is a C₄ to C₁₂ alkane. In embodiments, the haloalkane is selected from C₂ to C₁₀ haloalkanes. The haloalkanes can comprise one or more halogen atoms selected from chlorine and fluorine. The alkyl or haloalkyl groups in the alcohols, dialkyl ethers, alkanes and haloalkanes can be linear, branched or cyclic. One or a mixture of one or more blowing agents can be used.

In embodiments, for environmental reasons, the one or more blowing agents are selected from alcohols, alkyl ethers and alkanes, and in further embodiments the one or more blowing agents are selected from alcohols and alkanes, and preferably are alcohols. Haloalkanes are preferably avoided, due to their potential ozone depletion properties, and also due to their generally higher global warming potential.

Examples of suitable blowing agents that can be used include ethanol, n-propanol, isopropanol, n-butanol, tert-butyl alcohol, n-pentanol, isopentanol, n-hexanol, isohexanol, heptanol, isoheptanol, octanol, isooctanol, tert-butyl acetate, butyl acetate, methyl tert-butyl ether, n-pentane, isopentane, neopentane, cyclopentane, cyclohexane, n-butane, isobutane, isohexane, neohexane, heptane, isoheptane, octane, isooctane, isodecane, and isododecane. In preferred embodiments, the blowing agent is selected from C₂ to C₈ alcohols, such as C₂ to C₆ alcohols or C₂ to C₄ alcohols. In further preferred embodiments, the blowing agent comprises a blowing agent selected from isooctane, isohexane, tert-butyl acetate, butyl acetate, methyl tert-butyl ether, tert-butyl alcohol, and combinations thereof. In particularly preferred embodiments the blowing agent comprises tert-butyl alcohol or isooctane or a combination thereof.

In the expandable microspheres, the one or more blowing agents are typically present in an amount of from 5 to 50 wt%, based on the total weight of isolated lignin and blowing agent(s), for example in the range of from 5 to 45 wt%, or from 10 to 40 wt%.

The expandable microspheres of the invention are obtainable by a spray drying process comprising mixing the isolated lignin, a solvent, and the blowing agent and then spraying the thus obtained mixture into a drying equipment to produce the thermally expandable microspheres having a polymeric shell surrounding a hollow core, in which the polymeric shell comprises the isolated lignin, and the hollow core comprises the blowing agent.

In principle, the spray drying equipment for performing the spray drying process is not limited and any conventional and commercially available spray drying equipment can be used for the spray drying process. A typical spray drying equipment suitable for the process described herein comprises a drying chamber equipped with a nozzle, an inlet for drying gas and an outlet which connects the drying chamber with a cyclone. Through the nozzle which is normally located at the top of the spraying chamber (but may be also located on any other portion of the spray dryer) the liquid to be atomized is sprayed, usually in combination with a spray gas, into the drying chamber. In the drying chamber, the atomized liquid is dried by the drying gas which is fed into the spraying chamber through the inlet for drying gas. The inlet of drying gas may for instance be located besides the nozzle. The atomized liquid dries and forms particles. The thus obtained particles are then fed together with the drying gas through the outlet of the drying chamber which is normally located in the bottom area of the drying chamber into a cyclone. In the cyclone the particles are separated from the drying air. The drying air may be further filtered to remove any residual particles from the drying air.

A suitable spray drying equipment for performing the spray drying process is the Büchi mini spry dryer B-290 which is commercially available from Büchi/Switzerland.

The order of adding the isolated lignin, the solvent, the blowing agent and, optionally, the further polymeric component is not restricted and any order can be chosen.

However, in a preferred embodiment, in the process for producing the expandable microspheres, the isolated lignin is mixed first with the solvent and, optionally, the further polymeric component, and then, in a further step, the blowing agent is added to the mixture.

The mixing of the isolated lignin can be carried out at ambient temperature, although temperatures in the range of from 5 to 75°C can be used. Mixing is usually performed till the isolated lignin has completely dissolved in the solvent.

In embodiments, the mixture of the isolated lignin with the solvent and, optionally, the further polymeric component, can be left or stirred for a period of time, for example from 1 to 100 hours, or from 2 to 50 hours. This can be at temperatures in the range of from 10 to 95°C, for example at a temperature of from 20 to 90°C.

In a further step, the blowing agent is added to the mixture of the isolated lignin, solvent, and optionally, the further polymeric component. Also this mixing step can be carried out at ambient temperature, although temperatures in the range of from 5 to 75°C can be used. Also this mixing step is usually performed till the blowing agent has completely dissolved in the solvent.

After the addition of the blowing agent to the mixture of the isolated lignin, solvent, and, optionally, the further polymeric component, the thus obtained mixture may be further stirred for a period of time, for example from 1 to 100 hours, or from 2 to 50 hours. Also this can be at temperatures in the range of from 10 to 95°C, for example at a temperature of from 20 to 90°C.

The mixture comprising the isolated lignin, the solvent, the blowing agent and, optionally, the further polymeric component is then sprayed into a drying equipment to produce the thermally expandable microspheres as described herein. The drying equipment may be a spray drying equipment as described above.

The optional spray gas that is sprayed through the nozzle together with the liquid to be atomized is not particularly limited and may be any suitable spray gas known by the skilled person. For instance, the spray gas may be selected from nitrogen, carbon dioxide, (pressurized) air, noble gases, such as argon, etc. Preferably, in the method for producing expandable microspheres as described herein a spray gas is used and more preferably this spray gas is nitrogen.

Also the drying gas is not particularly limited and may be any suitable drying gas known by the skilled person. For instance, also the drying gas may be selected from nitrogen, carbon dioxide, (pressurized) air, noble gases, such as argon, etc. Preferably, the drying gas is nitrogen.

Further process parameters for running the spray drying equipment, such as the spray gas flow, the inlet temperature of the drying gas when entering the drying chamber, the feed rate of the liquid to be atomized and the aspirator speed to circulate the drying gas in the spray drying equipment can be readily chosen by the skilled person.

It has been found that with the above-described method it is possible to obtain expandable microspheres comprising a polymeric shell surrounding a hollow core, wherein the hollow core comprises a blowing agent, and the polymeric shell comprises isolated lignin.

The solvent can be water or an organic solvent selected from those having one or more functional groups selected from esters, amides, aldehydes, ketones, alcohols (including glycols) and ethers, for example those having 3 to 12 carbon atoms. Esters, ketones and ethers may, in embodiments, be part of a cyclic structure. Further examples include haloalkanes having from 1 to 6 carbon atoms and halo-carboxylic acids having from 1 to 6 carbon atoms, where the halogen is selected from fluorine, chlorine, bromine and iodine. The solvent can also be a mixture of water and an organic solvent, such as any of the organic solvents as described above.

Examples of organic solvents that can be used include ethyl acetate, ethyl formate, methyl acetate, n-propyl formate, isopropyl formate, n-propyl acetate, isopropyl acetate, isobutyl acetate, n-butyl acetate, n-pentyl formate, isopentyl formate, n-pentyl acetate, isopentyl acetate, ethyl propionate, isobutyl isobutyrate, n-butyl propionate, ethyl 3-ethoxypropionate, 2-ethylhexyl acetate, acetone, methyl ethyl ketone, diethyl ketone, methyl isobutyl ketone, methyl isoamyl ketone, methyl n-amyl ketone, mesityl oxide, acetophenone, cyclohexanone, diethyl phthalate, ethyl lactate, benzyl acetate, butyrolactone, acetyl acetone, methyl cyclohexanone, benzaldehyde, diisobutyl ketone diacetone alcohol, ethylene glycol, glyceryl-α-monochlorohydrin, propylene glycol, glycol ethers (for example propylene glycol monomethyl ether, ethylene glycol mono-methyl ether, ethylene glycol mono-ethyl ether, ethylene glycol mono-n-butyl ether, propylene glycol mono-tert-butyl ether, propylene glycol monopropyl ether, propylene glycol monobutyl ether), glycol ether esters (for example ethylene glycol mono-methyl ether acetate, ethylene glycol mono-ethyl ether acetate, ethylene glycol mono-butyl ether acetate, ethylene glycol diacetate), n-propyl alcohol, isopropyl alcohol, n-butanol, sec-butanol, isobutanol, benzyl alcohol, diisopropyl ether, dimethoxymethane, dimethoxyethane, 1,4-dioxane, 1,3-dioxolane, tetrahydrofuran, anisole, phenetole and dimethyl formamide. Other examples of solvents include dimethyl sulfoxide, toluene, xylene, n-methyl-2-pyrrolidone, methylene chloride, chloroform, carbon tetrachloride, trichloroacetic acid, methylene bromide, methylene iodide, trichloroethylene, and tetrachloroethylene. The organic solvent can be a mixture of two or more solvents. If the solvent is a mixture of two or more organic solvents, it is preferred that one solvent thereof is acetone. In embodiments, the acetone is mixed with an alcohol, such as methanol or ethanol.

In some embodiments, the solvent is a mixture of water and an organic solvent. In such embodiments, it is preferred that the mixture contains water in an amount of not more than 50 wt%, such as not more than 40 wt%, not more than 30 wt%, or not more than 20 wt% of water. In such embodiments, it is further preferred that the mixture contains water in an amount of at least 1 wt%, such as at least 2 wt% or at least 5 wt%, the wt% being based on the total weight of the solvent.

In particularly preferred embodiments, the solvent is selected from one or more of water, methanol, ethanol, and acetone. More preferred is that the solvent comprises water, methanol, ethanol, or acetone. In some particular preferred embodiments, the solvent comprises acetone. In some embodiments, the solvent comprises a mixture of at least two solvents, which are preferably selected from the group consisting of water, methanol, ethanol, acetone, and combinations thereof. In some embodiments, the solvent comprises acetone and water, or acetone and ethanol.

Typically, the isolated lignin content in the mixture for spray drying is typically in the range of from 0.1 to 50 wt%. In embodiments, it can be in the range of from 1 to 40 wt%, for example in the range of from 5 to 35 wt% or even from 10 to 30 wt%. The wt% are based on the total weight of the mixture for spray drying.

The amount of blowing agent(s) in the mixture for spray drying is typically in the range of from 0.5 to 50 wt%. In embodiments, it can be in the range of from 0.5 to 40 wt%, for example in the range of from 1 to 30 wt%, from 3 to 25 wt%, or even from 5 to 25 wt%. In embodiments, the weight of blowing agent in the mixture for spray drying is equal to or less than the weight of isolated lignin, for example the weight ratio of blowing agent to isolated lignin can be 1.5 or less, for example 1.3 or less or even 1.1 or less. In embodiments the minimum weight ratio is 0.1, or in further embodiments 0.2. In embodiments, the weight ratio of blowing agent to isolated lignin in the solvent phase is in the range of from 0.1 to 1.5, such as in the range of from 0.2 to 1.3 or even from 0.3 to 1.1.

The amount of the solvent adds up to 100 wt%. Preferably, the amount of organic solvent is at least 30 wt%, more preferably at least 40 wt% and even more preferably at least 50 wt%. The wt% are based on the total weight of the mixture for spray drying.

The unexpanded microspheres typically have volume mean particle sizes (diameters), i.e. D(0.5) values, in the range of from 1 to 500 µm, such as 5 to 200 µm or, in embodiments, from 10 to 100 µm or even from 30 to 80 µm.

The expanded microspheres are typically in the range of from 1.5 to 8 times larger in diameter than unexpanded microspheres, for example 2 to 7 times or 3 to 6 times their original diameter.

The particle sizes are suitably measured using light scattering techniques, e.g. laser diffraction, such as low angle laser light scattering (LALLS). They can also be measured by image analysis from a photograph or electronic micrograph image of the pre- or post-expanded microspheres.

To expand the expandable microspheres, they can be heated to a temperature above the boiling point of the blowing agent and the T_{g} of the isolated lignin, and also a temperature below the melting point of the microspheres. To halt the expansion, the microspheres can be cooled back down to below the T_{g} of the isolated lignin and/or the boiling point of the blowing agent.

Ways of heating the expandable microspheres include direct or indirect contact with a heat transfer medium such as steam or pressurised steam, as described for example in WO2004/056549, WO2014/198532 and WO2016/091847. In further embodiments, direct or indirect contact with other heated gases (e.g. air or nitrogen) optionally mixed with steam can be used. In still further embodiments, where indirect heating is used, a liquid heat transfer medium can be used, e.g. heated oil. In another embodiment, IR radiation can be used to heat the microspheres.

Expansion properties of the thermally expandable thermoplastic microspheres can be evaluated using a thermomechanical analyser (e.g. a Mettler TMA 841) and quantitative data can be obtained from images using suitable software, for example STARe software.

The expandable thermoplastic microspheres may be provided in unexpanded form, e.g. for expansion local to their point of use, or they can be pre-expanded before dispatch to the point of end use.

The microspheres can find use in many applications, for example in the manufacture of paper (e.g. embossed paper, a paper filler, a sizing agent), inks, corks, cement based compositions, adhesives, foams, insulation materials, coatings, rubber-based products, thermoplastics, thermosets, ceramics, non-woven composite materials, fillers etc. to provide for example a lightweight filler in such applications.

The expansion of the microspheres is typically irreversible, i.e. cooling the microspheres after thermal expansion does not result in their contraction back to their pre-expanded size.

Another aspect of the present invention is a process for preparing thermally expandable microspheres, the process comprising mixing an isolated lignin, a solvent, a blowing agent and, optionally, the further polymeric component, and then spraying the thus obtained mixture into a drying equipment to produce the thermally expandable microspheres having a polymeric shell surrounding a hollow core, in which the polymeric shell comprises the isolated lignin, and the hollow core comprises the blowing agent.

The process parameters, the spray drying equipment, the isolated lignin, the solvent, the blowing agent, the optional further polymeric component, as well as amounts thereof are the same as already described above and equally apply for the process according to the second aspect of the present invention.

In a further aspect, the present invention is also directed to thermally expandable microspheres obtained by the process for preparing thermally expandable microspheres as described above.

### Examples

The following examples are intended to illustrate the invention.

Microscopic evaluation of the expansion properties was performed using a Linkam LTS420 heating stage in combination with a Leica DM1000 microscope. The heating rate was 40°C/min.

The expansion characteristics were evaluated using a Mettler TMA 841 thermomechanical analyser, interfaced with a PC running STARe software. The heating rate was 20°C/min, using a load (net.) of 0.06 N.

Gas chromatography - flame-ionization detection (GC-FID) analyses have been performed using an Agilent 7697A Headspace in combination with an Agilent 7890A GC.

### General Synthesis Method:

A solution of isolated lignin, a blowing agent and, if applicable, a further polymeric component in a suitable solvent was prepared by dissolving the polymer, the blowing agent and, if applicable, a further polymeric component overnight with the use of a magnetic stirrer.

The further polymeric components were: PEG (polyethylene glycol; obtained from Merck, 807483); PAA (polyacrylic acid; molecular weight 2.000; 63%; obtained from VWR, ACRO184992500); HC (hemicellulose; obtained from IRIS Biotech, Xylan GBB1670); CMC (carboxymethyl cellulose; molecular weight 8.000-10.000 (GPC, Pullulan standards); DS 0.7; obtained from Nouryon); CA (cellulose acetate; obtained from Eastman, CA-398-3); chitosan (obtained from Chitolytic, CO-101211); and CAPh (cellulose acetate phthalate; obtained from Merck, 22192).

The thus obtained mixture was then sprayed-dried using a Büchi Mini Spray Dryer B-290. Nitrogen was used as spray gas with a feed rate of 238 I/h. The feed rate of the mixture to be spray dried was 4-13 ml/min (depending on the solvent). The temperature of the drying gas at the inlet was 70-120°C (depending on the solvent) and the aspirator rate was 38 m³/h.

Dried solids were collected from the bottom of the cyclone and analysed.

Table 1 lists the isolated lignin polymers that were used to prepare microspheres.

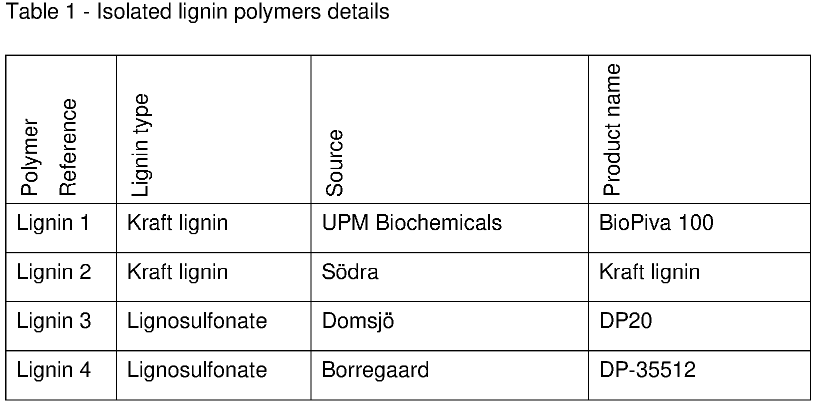

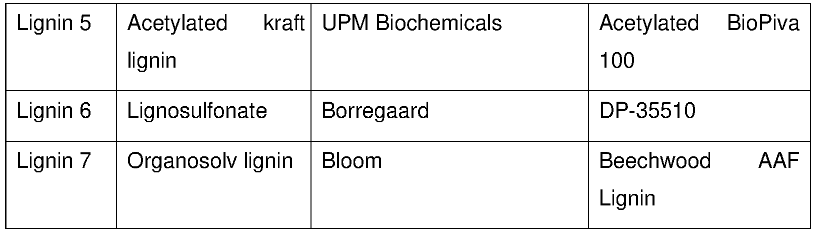

Details of the samples that were prepared are shown in Table 2, and data on the resulting microspheres are shown in Table 3. In each of Examples 13-17, the weight ratio (w/w) of the polymer to the further polymeric compound was 80/20. In Examples 6 and 9, the weight ratio (w/w) of the polymer to the further polymeric compound was 90/10. In Example 18, the weight ratio (w/w) of the polymer to the further polymeric compound was 97/3. In Examples 7 and 19, the weight ratio (w/w) of the polymer to the further polymeric compound was 95/5.

**Table 2 - Synthesis Details**

| Example | Polymer (wt%) (1) | Solvent (wt%) (1) (2) | Blowing Agent (wt%) (1) (3) | Further Polymeric Component (wt%) (1) |
|---|---|---|---|---|
| 1 | Lignin 1 (23.5 wt%) | Actn/water (ratio 90:10) (70.6 wt%) | t-BuOH (5.9 wt%) | None |
| 2 | Lignin 1 (23.5 wt%) | Actn/methanol (ratio 80:20) (70.6 wt%) | t-BuOH (5.9 wt%) | None |
| 3 | Lignin 1 (23.5 wt%) | Actn/ethanol (ratio 80:20) (70.6 wt%) | t-BuOH (5.9 wt%) | None |
| 4 | Lignin 2 (22.4 wt%) | Actn/water (ratio 90:10) (72.5 wt%) | t-BuOH (5.0 wt%) | None |
| 5 | Lignin 3 (28.6 wt%) | Water (64.3 wt%) | t-BuOH (7.1 wt%) | None |
| 6 | Lignin 3 (27.7 wt%) | Water (62.3 wt%) | t-BuOH (6.9 wt%) | PEG (4) (3.1 wt%) |
| 7 | Lignin 3 (27.1 wt%) | Water (64.3 wt%) | t-BuOH (7.1 wt%) | PAA (5) (1.4 wt%) |
| 8 | Lignin 4 (28.6 wt%) | Water (64.3 wt%) | t-BuOH (7.1 wt%) | None |
| 9 | Lignin 4 (27.7 wt%) | Water (62.3 wt%) | t-BuOH (6.9 wt%) | PEG (4) (3.1 wt%) |
| 10 | Lignin 5 (22.2 wt%) | Actn (66.7 wt%) | t-BuOH (11.1 wt%) | None |
| 11 | Lignin 6 (28.6 wt%) | Water (64.3 wt%) | t-BuOH (7.1 wt%) | None |
| 12 | Lignin 7 (20.8 wt%) | Actn/water (ratio 90:10) (75.0 wt%) | t-BuOH (4.2 wt%) | None |
| 13 | Lignin 3 / (22.9) | Water (64.3) | t-BuOH (7.1) | HC (6) (5.7) |
| 14 | Lignin 3 (22.9) | Water (64.3) | t-BuOH (7.1) | CMC (7) (5.7) |
| 15 | Lignin 3 (22.9) | Water (64.3) | t-BuOH (7.1) | Chitosan (5.7) |
| 16 | Lignin 1 (18.8) | Actn/water (ratio 80:20) (70.6) | t-BuOH (5.9) | CA (8) (4.7) |
| 17 | Lignin 1 (18.8) | Actn/water (ratio 80:20) (70.6) | t-BuOH (5.9) | CAPh (9) (4.7) |
| 18 | Lignin 1 (22.9) | Actn/water (ratio 90:10) (70.6) | t-BuOH (5.9) | Chitosan (0.6) |
| 19 | Lignin 1 (22.4) | Actn/water (ratio 90:10) (70.6) | t-BuOH (5.9) | Chitosan (1.2) |

| | | | | |
|---|---|---|---|---|
| (1) wt% based on the total weight of the mixture (2) Actn = acetone (3) t-BuOH = tert-butyl alcohol (4) PEG = polyethylene glycol (5) PAA = polyacrylic acid (6) HC = hemicellulose (7) CMC = carboxymethyl cellulose (8) CA = cellulose acetate (9) CAPh = cellulose acetate phthalate | | | | |

**Table 3 - Microsphere Properties**

| Example | Expanded Density / g dm⁻³ | Blowing Agent Content / wt% (1) | Tₛₜₐᵣₜ / °C (2) | Tₘₐₓ / °C (3) | Microscopic Evaluation (4) |
|---|---|---|---|---|---|
| 1 | 129 | 10.2 | 129 | 155 | +++ |
| 2 | 167 | 10.3 | 131 | 153 | +++ |
| 3 | 199 | 10.2 | 132 | 152 | ++ |
| 4 | 156 | 9.3 | 144 | 164 | ++ |
| 5 | 727 | - | 179 | 192 | + |
| 6 | 496 | - | 131 | 157 | ++ |
| 7 | 408 | - | 174 | 214 | ++ |
| 8 | 192 | - | 182 | 205 | ++ |
| 9 | 247 | - | 137 | 162 | ++ |
| 10 | - | - | - | - | + |
| 11 | - | - | - | - | + |
| 12 | - | 8.7 | - | - | + |
| 13 | 182 | - | 181 | 214 | ++ |
| 14 | 466 | - | 184 | 199 | + |
| 15 | 512 | - | 188 | 238 | + |
| 16 | 175 | 9.2 | 138 | 184 | ++ |
| 17 | 162 | 8.8 | 137 | 170 | ++ |
| 18 | 174 | 10.2 | 133 | 155 | ++ |
| 19 | 259 | 10.4 | 134 | 191 | ++ |

| | | | | | |
|---|---|---|---|---|---|
| (1) Blowing agent content of the microspheres, measured by GC-FID (2) Temperature at which microspheres began to expand (3) Temperature at which maximum microsphere expansion observed (4) Microscopic evaluation of the expansion properties of unexpanded microspheres; with + = good; ++ = very good +++ = excellent - = not determined | | | | | |

These data reveal that the isolated lignin compositions can be used to make bio-based microspheres having at least a good expansion under microscopic evaluation and which have a high versatility as regards adjustable temperatures at which expansion starts over a wide range. Moreover, the microspheres of the present invention have suitable expanded densities for use in a wide range of applications and, hence, have a desirable expansion performance. Even further, it has been shown that expandable microspheres can also be prepared based on mixtures of lignin polymer and a wide variety of further polymeric compounds, including other bio-based polymeric compounds.

### Flammability Tests:

For testing the flammability of expandable microspheres, about 15 ml of expandable microspheres were gathered in a long string of about 10 cm length with slanting sides. One end of the string was then set on fire, and the time for the flame to traverse the entire string (i.e., travel from one end of the string to the other) was recorded. Tests were performed on the microspheres of Examples 1-3 and 5. Commercially available microspheres, available as Expancel FG41 and 920DU40 (obtained by suspension polymerization of fossil based ethylenically unsaturated monomers), were used as a reference. The strings formed from the inventive Kraft lignin and lignosulfonate microspheres (Examples 1-3 and 5, respectively) almost immediately self-extinguished (i.e., the flame was extinguished by the microspheres before it had a chance to traverse the string to any notable extent, thus demonstrating the fire retardant properties of the microspheres) whereas the comparative microspheres Expancel FG41 and 920DU40 took respectively 105 and 195 seconds to burn completely (i.e., those microspheres did not possess fire retardant properties).

## Claims

1. Thermally expandable microspheres comprising a polymeric shell surrounding a hollow core, wherein the hollow core comprises a blowing agent, and the polymeric shell comprises isolated lignin.

2. Thermally expandable microspheres according to claim 1, wherein the isolated lignin comprises chemically modified lignin.

3. Thermally expandable microspheres according to claim 1 or 2, wherein the isolated lignin comprises a lignin selected from the groups consisting of Kraft lignin, organosolv lignin, sulfonated lignin, hydrolysis lignin, steam explosion lignin, milled wood lignin, soda lignin, and combinations thereof, and preferably is sulfonated lignin, Kraft lignin, or a combination thereof.

4. Thermally expandable microspheres according to any of claims 1-3, wherein the isolated lignin comprises Kraft lignin, such as acetylated Kraft lignin.

5. Thermally expandable microspheres according to any of claims 1-4, wherein the polymeric shell comprises up to 50 wt%, preferably 1 to 45 wt%, more preferably 5 to 40 wt%, of at least one further polymeric component, wherein the wt% are based on the total weight of the polymer blend of the polymeric shell.

6. Thermally expandable microspheres according to any of claims 1-5, wherein the polymeric shell further comprises a further polymeric component selected from the group consisting of polysaccharides, polysaccharide derivatives, polyesters, polyethers, polyacids, polyols, polyalkenes, or a combination thereof.

7. Thermally expandable microspheres according to claim 6, wherein the further polymeric component is selected from the group consisting of alkyl cellulose, carboxy cellulose, copolymers of vinyl acetate, polylactic acid, polyethylene glycol, polyacrylic acid, and combinations thereof.

8. Thermally expandable microspheres according to claim 6 or 7, wherein the further polymeric component is selected from the group consisting of polyethylene glycol, polyacrylic acid, and combinations thereof.

9. Thermally expandable microspheres according to any of claims 1-8, wherein the blowing agent comprises an alcohol, an ether, an ester, or a hydrocarbon, preferably an alcohol or a hydrocarbon.

10. Thermally expandable microspheres according to any of claims 1-9, wherein the blowing agent comprises a blowing agent selected from the group consisting of isooctane, isohexane, tert-butyl acetate, butyl acetate, methyl tert-butyl ether, tert-butyl alcohol, and combinations thereof, and preferably comprises tert-butyl alcohol or isooctane.

11. A method for preparing thermally expandable microspheres comprising a polymeric shell surrounding a hollow core, wherein the hollow core comprises a blowing agent, and the polymeric shell comprises isolated lignin, the method comprising the following steps:
(i) preparing a mixture comprising the isolated lignin and the blowing agent in a solvent; and
(ii) spray-drying the mixture obtained in step (i) so to obtain thermally expandable microspheres.

12. The method of claim 11, wherein the solvent is selected from the group consisting of water, alcohols, ketones, ethers, esters, and combinations thereof.

13. The method of claim 11 or 12, wherein the solvent comprises a mixture of at least two solvents selected from the group consisting of water, methanol, ethanol, acetone, and combinations thereof.

14. Use of thermally expandable microspheres according to any one of claims 1-10 as a fire retardant material.
